(19) **Europäisches Patentamt / European Patent Office / Office européen des brevets**

(11) **EP 1 764 035 B1**

(12) **EUROPEAN PATENT SPECIFICATION**

(45) Date of publication and mention
of the grant of the patent:
**13.06.2012 Bulletin 2012/24**

(51) Int Cl.:
***A61B 5/08*** (2006.01)

(21) Application number: **06019342.2**

(22) Date of filing: **15.09.2006**

(54) **Device for the measurement of single-breath diffusing capacity (DLco) of the lung using ultrasound molar mass measurement**

Gerät zur Messung der Diffusionskapazität der Lunge für einen Atemzug mittels Ultraschallmessung der molaren Masse

Outil pour la détermination de la capacité de la diffusion pulmonaire pour une inspiration utilisant la mésure ultrasonore de la masse molaire

(84) Designated Contracting States:
**AT BE BG CH CY CZ DE DK EE ES FI FR GB GR HU IE IS IT LI LT LU LV MC NL PL PT RO SE SI SK TR**

(30) Priority: **16.09.2005 US 717643 P**

(43) Date of publication of application:
**21.03.2007 Bulletin 2007/12**

(73) Proprietor: **ndd Medizintechnik AG 8005 Zürich (CH)**

(72) Inventors:
• **Buess, Christian 8810 Horgen (CH)**
• **Harnoncourt, Georg 8008 Zürich (CH)**
• **Kleinhappl, Erich 8810 Waedenswill (CH)**

(74) Representative: **Laufhütte, Dieter et al Lorenz-Seidler-Gossel Widenmayerstrasse 23 80538 München (DE)**

(56) References cited:
**EP-A- 1 632 178       EP-A- 1 764 036**
**EP-A2- 1 112 716     WO-A-02/24070**
**US-A- 5 022 406       US-A- 5 645 071**

## Description

Measurement of $DL_{CO}$ is common practice in most lung function laboratories. A $DL_{CO}$ quantifies the diffusion rate of gas into the blood. Since the diffusion rate of oxygen cannot be measured directly, carbon monoxide (CO) is used. In order to perform a $DL_{CO}$ measurement a gas mixture with two test gases is used. With the first test gas (CO) the diffusion rate is measured. With the second test gas the alveolar volume is measured. For that purpose several inert gases can be used. Most implementations either use helium (He) or $CH_4$. The single breath $DL_{CO}$ test procedure, which is standardized by the American Thoracic Society [5], requires the patient to perform a full inhalation of test gas. A commonly used test gas mixture is 0.3% CO, 10% He, 21 % $O_2$ in $N_2$. Since CO is toxic the level of CO must be very low. After the full inhalation the patient must perform a breath hold for approx. 10 seconds. During that time the CO diffuses into the blood and the inert gas (e.g. He) equilibrates within the lungs. The breath hold is followed by a rapid exhalation. During that exhalation the concentrations of He and CO are measured. The helium concentration measurement is used to compute the alveolar volume; the CO concentration is used to quantify diffusion. In- and expiratory volumes are measured by an appropriate flow sensor or a volume spirometer.

Most instruments that are currently in use are relatively large and require calibration of all sensors on a regular basis. In order to measure $DL_{CO}$ the following sensors must be available: A flow sensor or a volume spirometer for measuring in- and exhaled gas volumes, a CO sensor, and a sensor for the inert gas (e.g. a helium sensor). Normally the gas sensors are mounted in a side-stream arrangement, where a small fraction of test gas is taken from the main-stream and fed to the side-stream gas sensors. These gas sensors normally require calibration to be performed on a regular basis. In some systems the calibration is performed automatically before each measurement or before each measurement series.

US 5022 406 discloses a module for determining diffusing capacity of the lungs for corbon monoxide.

The valve system for the $DL_{CO}$ gas supply is another critical component in the test setup. The valve system must perform the following operations: 1) Allow the patient to breathe air freely at the beginning of the test, 2) instantaneously switch to $DL_{CO}$ gas mix on a clearly defined inspiration, 3) inhibit in- and expiration during the breath hold phase, 4) open and allow free expiration after the breath hold phase. Current systems use different types of valve systems, most systems are based on pneumatically controlled valves, so called 'balloon valves' or 'cushion valves'.

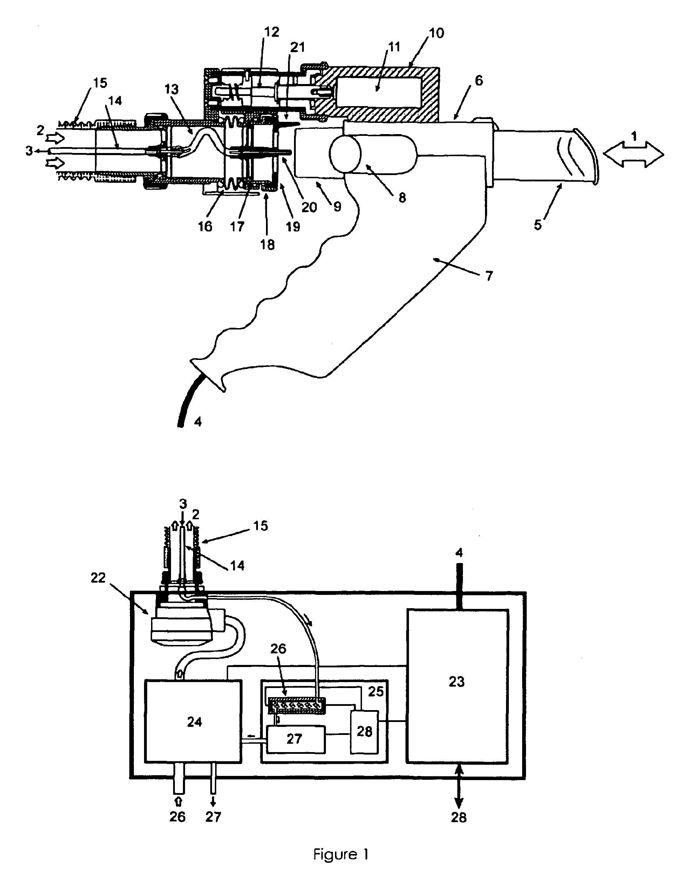

Figure 1 shows a schematic representation of the system for $DL_{CO}$ measurement. The top of the schematic shows the flow sensor with an electromechanically operated valve system. The data acquisition and control system is shown on the lower part.

The system is based on an ultrasonic flow sensor that is well described in literature [1,2,3,4]. The flow sensor consists of an ergonomic handle (7), enclosures for the ultrasonic transducers (8), a flow tube holder (6) and an exchangeable flow tube with mouth piece (5). On top of the flow sensor an electrical motor (11) is mounted in an appropriate housing (10). The axis of the motor is constructed in such a way that it can easily be mechanically disconnected from the valve system shown on the left side. Over its axis (12) the motor can actuate a sliding mechanism that moves the valve system towards the end of the flow tube (9) or away from it. In its 'closed' position part 19 (which is made out of soft rubber, e.g. silicone), is in direct contact to the end of the flow tube. Part 18 is a holder for a one-way valve 17 and part 19 that comes into contact with the flow tube. The one-way valve is made out of a soft rubber disc. The assembly of parts 17, 18 and 19 can easily be extracted from the valve system. This allows easy exchange or cleaning of those parts which come into direct contact with exhaled breath of the patient. Part 16 provides a flexible but air tight connection of the $DL_{CO}$ gas supply to the moving one-way valve. The $DL_{CO}$ gas is supplied by a standard flexible hose (15) connected to the valve system. Within the $DL_{CO}$ gas supply tube an additional small diameter tubing (14) is connected over a flexible part (13) to a tip (20) located at the center of the end of the flow tube (9). Over tip (20) gas is fed to the side-stream gas analyzers in the control system. Tube (14) may consist of normal plastic tubing or of special tubing that equilibrates water vapor.

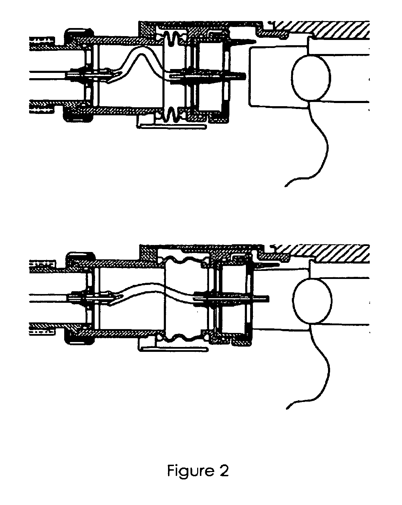

Figure 2 shows the valve system in the 'open' and in the 'close' position.

The entire system of flow sensor and valve assembly is lightweight and can be held by the patient or it can alternatively be mounted to an appropriate stand. $DL_{CO}$ test gas (2) is fed to the system over hose 15; side-stream gas (3) is fed to the analyzers over a small tubing (14); and the sensor is connected to the control system over a cable (4) containing all power and signals lines required to control both flow sensor and valve system.

The control system shown on the lower part of Figure 1 consists of a demand valve (22) that supplies $DL_{CO}$ test gas to the patient, a pneumatic subsystem (24), a side-stream gas analyzer subsystem (25) that analyzes the gas samples taken at the end of the flow tube, and a control unit (23). The control unit (23) connects to the pneumatic subsystem (24), to the side-stream gas analyzers subsystem (25) and to the flow sensor with integrated valve (shown in the upper part). The control unit actuates valves and controls the pump for the side-stream system, it samples data from the ultrasonic flow and molar mass sensor and it controls and samples data from the side-stream gas analyzer system. The system interfaces to an evaluation unit (e.g. a PC) over an interface (28); $DL_{CO}$ gas is supplied at input 26 and the side-stream gas exhausts over port 27.

Gas sampled at port 20 of the valve system is fed over tubes 13 and14 to the side-stream flow and molar mass sensor (26), then to the side-stream CO sensor (27) and finally to the pneumatic subsystem (24) where a pump is used to drive the side-stream flow.

The side-stream CO sensor system may additionally contain an $O_2$ and/or a $CO_2$ sensor in order to compensate cross sensitivity of the CO sensor to $O_2$ and/or $CO_2$. A $CO_2$ sensor may also be used to compensate the influence of $CO_2$ on the molar mass measurement used for the computation of the helium concentration.

In both main-stream and side-stream flow and molar mass sensors the gas flow is computed using the following equation:

$$F = k \frac{t_1 - t_2}{t_1 \cdot t_2}$$

where F is the velocity of the gas flow, $t_1$ and $t_2$ represent the transit-times in up-and downstream direction, and k is a constant that depends on the mechanical dimensions of the flow sensor.

Molar mass is computed using the following equation:

$$M = k \cdot \kappa \cdot R \cdot T \cdot \left( \frac{t_1 \cdot t_2}{t_1 + t_2} \right)^2 ,$$

where M is the molar mass, T is the mean temperature along the sound transmission path, R is the gas constant, $\kappa$ is the relation of the specific heat capacities ($c_p/c_v$) of the gas, k is a constant that depends on the mechanical dimensions of the sensor, and $t_1$ and $t_2$ represent the transit times (see [2]). Temperature T can be determined by one or several temperature measurements along the sound transmission path; it can be determined by a combination of a temperature measurement and a mathematical model; or it can be set to a constant value (e.g. to zero degree Celsius).

Due to its operating principle flow and molar mass signals of an ultrasonic flow sensor are time aligned. In order to perform the time alignment of the main-stream flow measurement and the side-stream molar mass measurement the technique described in [6] may be applied.

In order to perform a measurement of $DL_{CO}$ the following procedure is performed: Prior to the test hose 15 is flushed with $DL_{CO}$ test gas. The valve system is set to the 'open' position where the patient can breathe room air. The patient performs a deep exhalation. At the end of the exhalation the valve system closes. The patient performs a rapid deep inhalation of $DL_{CO}$ test gas. The patient performs a 10 second breath hold. Since the valve system is in its 'closed' position the patient cannot exhale during the breath hold time. At the end of the 10 second period the valve system opens and the patient exhales. During the entire test gas is sampled at port 20 and fed to the side-stream gas and molar mass sensors. Gas sampling may be temporarily stopped during the 10 second breath hold time.

In order to compute the $DL_{CO}$ value the following parameters are computed:

1. In- and expiratory volumes are determined from the flow signals of the ultrasonic flow sensor by integrating flow over time.

2. The CO gas concentration of the expiration after the breath hold is determined by the CO sensor. The CO sensor may be calibrated using inspiratory room air (0% CO) and the $DL_{CO}$ gas mixture that is available during the inspiration at the beginning of the test. In addition the CO sensor may be corrected for $O_2$ cross sensitivity by using the signal of an additional $O_2$ gas sensor.

3. The helium concentration of the expiration after the breath hold is determined by the side-stream ultrasonic molar mass sensor. Since molar mass does not directly determine the helium concentration the influences of the other gas components are considered using mathematical models and/or measurements of additional side-stream sensors for $O_2$ and/or $CO_2$.

**Claims**

1. Device for the measurement of the diffusing capacity ($DL_{CO}$) of the lung consisting of a main-stream flow sensor with attached electromechanically operated valve system configured the controlled delivery of $DL_{CO}$ test gas (2) to a patient, a side-stream molar mass sensor (26) configured for the computation of the tracer gas concentration required for the determination of the alveolar volume, and a side-stream CO sensor (27), **characterized in that** the main stream sensor is a
ultrasonic flow and molar mass sensor and the mainstream flow and molar mass sensor and side-stream flow and molar mass sensor (26) are configured to compute the gas flow by

$$F = k \frac{t_1 - t_2}{t_1 \cdot t_2} ,$$

where F is the velocity of the gas flow, $t_1$ and $t_2$ represent the transit-times in up- and downstream direction, and k is a constant that depends on the mechanical dimensions of the flow sensor, in which the device is configured to determine the time delay between main-stream flow signal and side-stream molar mass signal and/or side-stream gas sensor signal(s) by performing correlation of these signals to the main-stream molar mass measurement.

2. Device according to claim 1, where all parts of the system that come into direct contact with the patients exhaled breath, the flow tube (9) and the one-way valve (17) that delivers the $DL_{CO}$ test gas (2), can be exchanged for disposal or cleaning purposes.

3. Device according to claim 2, where the gas sampling port (20) and the one-way valve system (17) for $DL_{CO}$ gas delivery is realized in a single exchangeable part.

4. Device according to claim 4 1, where an additional side-stream $O_2$ sensor and/or an additional side-stream $CO_2$ sensor is used to correct a possible cross sensitivity of the CO sensor (27) to $O_2$ and/or $CO_2$.

5. Device according to claim 4, where a bacteria filter is mounted in front of the flow tube (9) in order to completely block cross contamination between patients.


**Patentansprüche**

1. Vorrichtung für die Messung der Diffusionskapazität ($DL_{CO}$) der Lunge bestehend aus einem Hauptstrom-Durchflusssensor mit angebrachtem, elektromechanisch betriebenen Ventilsystem, das für die gesteuerte Zufuhr von DLco-Testgas (2) zu einem Patienten konfiguriert ist, einem Nebenstrom-Molmasse-Sensor (26), der für die Berechnung der Tracergaskonzentration konfiguriert ist, die zur Ermittlung des alveolaren Volumens erforderlich ist, und einem Nebenstrom-CO-Sensor (27), **dadurch gekennzeichnet, dass** der Hauptstrom-Sensor ein Ultraschalldurchfluss- und Molmasse-Sensor ist und der Hauptstrom-Durchfluss- und Molmasse-Sensor und Nebenstrom-Durchfluss- und Molmasse-Sensor (26) zum Berechnen des Gasdurchflusses durch

$$F = k\frac{t1 - t2}{t1 \cdot t2}$$

konfiguriert sind, wobei F die Geschwindigkeit des Gasdurchflusses ist, $t_1$ und $t_2$ die Laufzeiten in Stromaufwärts- und Stromabwärtsrichtung darstellen und k eine Konstante ist, die von den mechanischen Maßen des Durchflusssensors abhängt, wobei die Vorrichtung konfiguriert ist, um die Zeitverzögerung zwischen dem Hauptstrom-Durchflusssignal und dem Nebenstrom-Molmasse-Signal und/oder Nebenstrom-Gassensorsignal(en) durch Vornehmen von Korrelation dieser Signale mit der Hauptstrom-Molmasse-Messung zu ermitteln.

2. Vorrichtung nach Anspruch 1, wobei alle Teile des Systems, die in direktem Kontakt mit dem ausgeatmeten Atem der Patienten, dem Durchflussschlauch (9) und dem Einwegventil (17), das das $DL_{CO}$-Testgas (2) zuführt, kommen, für Entsorgung oder Reinigungszwecke ausgewechselt werden können.

3. Vorrichtung nach Anspruch 2, wobei die Gasprobeentnahmeöffnung (20) und das Einwegventilsystem (17) für die DLco-Gaszufuhr in einem einzigen auswechselbaren Teil verwirklicht sind.

4. Vorrichtung nach Anspruch 1, wobei ein zusätzlicher Nebenstrom-$O_2$-Sensor und/oder ein zusätzlicher Nebenstrom $CO_2$-Sensor verwendet wird, um eine mögliche Querempfindlichkeit des CO-Sensors (27) auf $O_2$ und/oder $CO_2$ zu korrigieren.

5. Vorrichtung nach Anspruch 4, wobei ein Bakterienfilter vor dem Durchflussschlauch (9) montiert ist, um eine Kreuzkontamination zwischen Patienten vollständig zu unterbinden.


**Revendications**

1. Appareil de mesure de la capacité de diffusion ($DL_{CO}$) du poumon se composant d'un capteur de débit à courant principal avec un système de soupape fixé et fonctionnant électromécaniquement, configuré pour la livraison contrôlée de gaz d'essai $DL_{CO}$ (2) à un patient, d'un capteur de masse molaire à courant latéral (26) configuré pour le calcul de la concentration de gaz de traçage requise pour la détermination du volume alvéolaire, et d'un capteur de CO à courant latéral (27), **caractérisé en ce que** le capteur à courant principal est un capteur de masse molaire et de débit d'ultrasons et **en ce que** le capteur de débit à courant principal et de masse molaire et le capteur de débit à courant latéral et de masse molaire (26) sont configurés pour calculer le débit de gaz par

$$F = k \frac{t_1 - t_2}{t_1 \cdot t_2},$$

où F est la vitesse de débit de gaz, $t_1$ et $t_2$ représentent les temps de transit en direction amont et aval, et k est une constante qui dépend des dimensions mécaniques du capteur de débit, dans lequel l'appareil est configuré pour déterminer le retard de parcours entre le signal de débit à courant principal et le signal de masse molaire à courant latéral et/ou le(s) signal/signaux du capteur de gaz à courant latéral en effectuant la corrélation de ces signaux par rapport à la mesure de masse molaire à courant principal.

2. Appareil selon la revendication 1, où toutes les parties du système qui entrent en contact direct avec la respiration exhalée par les patients, le tube de débit (9) et la soupape unidirectionnelle (17) qui livre le gaz d'essai DLCO (2), peuvent être échangés à des fins de mise au rebut ou de nettoyage.

3. Appareil selon la revendication 2, où l'orifice d'échantillonnage de gaz (20) et le système à soupape unidirectionnelle (17) pour la livraison de gaz $DL_{CO}$ sont réalisés dans une partie échangeable individuelle.

4. Appareil selon la revendication 1, où un capteur supplémentaire de $O_2$ à courant latéral et/ou un capteur supplémentaire de $CO_2$ à courant latéral est utilisé pour corriger une sensibilité croisée possible du capteur de CO (27) au $O_2$ et/ou au $CO_2$.

5. Appareil selon la revendication 4, où un filtre à bactéries est monté devant le tube de débit (9) afin de bloquer complètement la contamination croisée entre les patients.

Figure 1

Figure 2

**EP 1 764 035 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5022406 A